# EUROPEAN PATENT SPECIFICATION

(11) **EP 3 638 368 B1**
(45) Date of publication and mention of the grant of the patent: **15.03.2023**
(21) Application number: 18735707.4
(22) Date of filing: 06.06.2018
(51) Int. Cl.: A61N 5/10

(54) **APPLICATOR DEVICE FOR INTERVENTION RADIOTHERAPY (BRACHYTHERAPY) AND PERINEAL INTERVENTION AND/OR DIAGNOSTIC PROCEDURES**
APPLIKATOR FÜR INTERVENTIONELLE RADIOTHERAPIE (BRACHYTHERAPIE) UND PERINEALINTERVENTION UND/ODER DIAGNOSTISCHE VERFAHREN
DISPOSITIF APPLICATEUR POUR RADIOTHÉRAPIE INTERVENTIONNELLE (CURIETHÉRAPIE) ET PROCÉDURES PÉRINÉALES INTERVENTIONNELLES ET/OU DE DIAGNOSTIC

(30) Priority: 16.06.2017 IT 201700067474
(43) Date of publication of application: 22.04.2020
(73) Proprietor: Università Cattolica del Sacro Cuore, 20123 Milano (IT)
(72) Inventor: VALENTINI, Vincenzo, 00168 Roma (IT); TAGLIAFERRI, Luca, 00168 Roma (IT)
(74) Representative: Manna, Sara
(86) International application number: PCT/IB2018/054038
(87) International publication number: WO 2018/229599

(56) References cited:
- WO-A1-2011/123605
- WO-A1-2016/009312
- US-A- 5 947 891
- US-A1- 2012 277 518
- US-A1- 2017 197 092

## Description

### Technical field of the invention

The present invention relates to an applicator device for intervention radiotherapy (brachytherapy) or for perineal intervention and/or diagnostic procedures, suitable to implement a guide for percutaneous insertion of:
- needles or catheters useful for carrying radioactive material temporarily or permanently;
- needles or other needle-like instruments for performing intervention/diagnostic procedures.

Moreover, the device has a shape so as to increase the accuracy in performing the procedures by means of using techniques for fusing images acquired with different diagnostic methods (Ex. MR and Ultrasound examination).

### Background

In the last years a quick evolution in the field of the intervention radiotherapy, better known as brachytherapy, has occurred.

Brachytherapy is a technique for treating tumour diseases by administrating radiations, which provides the (temporary or permanent) positioning of one or more radioactive sources inside or near the body district to be treated by means of an applicator device.

Generally, brachytherapy is used effectively in treating diseases affecting the pelvic area, such as cancer of prostate, vulva, anus, rectum and vagina.

In the last decade, the use of image survey techniques of the body districts to be treated, such as 3D *imaging* techniques, CT, magnetic resonance, PET/CT, ultrasound examination, has become more and more widespread, which techniques have led to the development of `Image Guided BrachyTherapy' (IGBT).

Such type of treatment allows to prepare increasingly customized therapeutic plans based upon the combination of images of the body district to be treated acquired by means of different techniques, as well as to simulate the execution of the treatment themselves.

Parallelly, even the apparatuses used in executing treatments have been improved, in particular miniaturized treatment apparatuses and mono-source apparatuses (*Remote After Loading* HDR and PDR apparatuses) have been implemented. The objective is to implement brachytherapy techniques with high precision control in administering radiations, to limit the useless exposition of organs at risk to the same (*Intensity Modulated BrachyTherapy,* IMBT).

The currently on the market applicators for brachytherapy allow to perform effectively the just described IGBT and IMBT techniques, but with some limitations.

As far as IMBT is concerned, it is necessary to have available applicators allowing to perform implants with high stability.

On the contrary, within IGBT, the plants for the image acquisition of the patient's body district according to the different illustrated techniques (resonance, ultrasound examination, and so on) provide hardly comparable images as the local anatomy of the soft tissues varies during each acquisition, due to the different used equipment. For example, by inserting a rectal probe for ultrasound examination, the anatomy of rectum and anus modifies as these organs are dilated by the probe itself, whereas during a CT or MR these organs appear with an anatomically normal size and position. This involves a low correspondence or *match* of the obtained images, therefore it is difficult to overlap them and process them with the purpose of obtaining results useful for preparing therapies or diagnostic procedures. Relevant prior art can be found in US2012/277518, US5947891 and WO2016/009312.

### Summary of the invention

The technical problem placed and solved by the present invention then is to provide a device which allows obviating the drawbacks mentioned above with reference to the known art.

The above-mentioned drawbacks are solved by an applicator device according to claim 1 and by an applicator assembly according to claim 9.

Preferred features of the present invention are set forth in the dependent claims.

The present invention provides an applicator device suitable to implement a guide for the percutaneous insertion of catheters, needles or more generally needle-shaped elements, for example bearing seeds of radioactive material to be deposited in a patient's body district or to collect tissue useful for diagnostic surveys.

The applicator device, or more simply applicator, can be used during the execution of diagnosis activities and treatment of pelvic pathologies, such as tumour of anus, rectum, prostate, vagina, vulva, for example by means of brachytherapy or other therapies. To this purpose, the applicator is wholly made of not metallic material, or however a material which does not shield radiations and which does not create artefacts during the diagnostic procedures, preferably plastic material. Therefore, it is possible to use it within procedures and MR and CT wholly safely for the patient.

The applicator comprises a substantially plate-shaped main body, that is having a very reduced size in space (that of thickness) with respect to the other two sizes (width and length), which define an operating surface bearing indeed the through holes implementing the guide for the percutaneous insertion of the needles or catheters.

Such main body has two curved, in particular concave - that is receding towards the inside of the main body - peripheral portions, shaped to adapt to the anatomy of the body district to be treated and to implement a stable positioning of the device during the treatment. In particular, the concavity is shaped to follow the anatomical course of the thigh origin, for applications at the front or rear pelvic area.

According to preferred embodiments of the invention device, the applicator has an overall T-like configuration, in particular in use the device is used according to an overturned T arrangement.

According to such configuration, the T shape provides to the applicator a great use versatility, as it is useful indistinctly for applications at anus, rectum, vulva or vagina, that is at body districts belonging to the front or rear pelvic region. Advantageously, thanks to the T shape it is possible to guide simultaneously needle-like elements or catheters to cover the whole treatment surface of all above-mentioned organs by means of one single device.

In particular, the portion at the T arms corresponds to a region of the applicator mainly responsible for the treatment of vulva, vaginal, anal and rectal cancers, whereas the portion at the T stem corresponds to a region mainly responsible for the treatment of prostate.

The applicator device has an operating surface, generally orthogonal to the direction wherein the plate thickness develops, which plate preferably is flat.

Such operating surface has a plurality of through holes, which develop aligned mutually along the whole thickness of the plate, preferably according to a direction orthogonal to the operating surface, configured to guide the percutaneous penetration of needles or catheters. The holes on the operating surface can be mutually equidistant, preferably arranged according to the vertexes of squares or equilateral triangles.

The applicator further has a through seat configured for inserting (and preferably for locking in an inserted position) a tubular guiding element, in particular an endo-rectal cylinder.

The tubular element implements a guide for performing several procedures, such as therapeutic interventions and diagnostic procedures providing the acquisition of images of the body district to be treated.

In particular, the endo-rectal cylinder is configured to receive inside thereof and guide a probe or other imaging device, for example ultrasound examination, inside a body district. To this purpose, the endo-rectal cylinder is preferably made of eco-transparent material.

Advantageously, the help of such tubular guiding element during the image acquisition, apart from stabilizing the device when in use, allows to cause the same deformation of the anatomy of soft tissues during the different procedures, by allowing to obtain images the match thereof (image fusion) is simple to be followed.

The seat apt to receive the tubular element preferably is arranged at a central portion of the operating surface, in particular it is centred with respect to the two concave portions of peripheral edge.

According to the invention, the holes for inserting the needles or catheters are distributed with homogeneous density at the peripheral portions of the operating surface, whereas the density increases around the surface central portion, in particular at the seat for inserting the endo-rectal cylinder.

The greater density of holes allows to insert a greater number of needles around the area subjected to the treatment (anus, vulva, vagina, and so on), for modulating more precisely the distribution of the radiation dose to be administered to the tumour target. Some holes of the area having greater density of holes, above all at the seat for inserting the endo-rectal cylinder, due to geometrical reasons could not be arranged in mutually equidistant positions.

A still additional preferred embodiment of the invention comprises a system for locking by friction the needles inserted in the applicator.

Such system provides that the applicator is implemented modularly, by superimposing and assembling several substantially plate-like elements, to implement a sandwich-like overall configuration.

The modular applicator comprises two outer bodies, made of stiff material (that is which cannot be perforated by the needles/catheters) and an intermediate body which can be interposed therebetween, made of soft material (that is which can be perforated by the needles/catheters).

Both outer bodies have a plurality of through holes for the insertion of the needles, which match in the configuration for assembling the applicator to allow the simultaneous sliding of the needles through both outer bodies.

In particular, the two outer bodies abut on each other to lock therebetween under compression the intermediate body, preferably reversibly, for example by clamping threaded elements (for example plastic screws, or however elements made of not metallic material).

In use, each needle-like element is inserted in a respective hole at the operating surface of an outer body, penetrates through the intermediate body and at last it outgoes from the opposite face of the applicator by means of a corresponding through hole through the additional outer body.

When the needle-like elements are inserted in the applicator, after application of a force so as to overcome the penetration resistance of the intermediate element, the soft material implements an obstacle to additional accidental motions of the same needle-like elements in the hole developing direction.

Additional preferred feature of an applicator according to the present invention is the presence of seats for anchoring the device itself to the patient's body district or to outer devices, for example elements for supporting imaging apparatuses (supports for ultrasound apparatus) or stepper.

Such anchoring seats can be implemented in the form of through holes, rings or eyelets configured for the application of stitches (for connecting the applicator to the patient's skin) or fixing screws (for connecting the applicator to devices or outer apparatuses), positioned on the peripheral profile of the applicator, in particular at end vertexes.

Advantageously, thanks to such anchoring seats the applicator can be hooked to steppers for ultrasound apparatuses, or directly to the patient's skin through stitches.

Preferably, some seats implemented as through holes are configured to allow, however, the insertion of a needle or catheter inside thereof.

Preferably, the screws or mechanisms for anchoring between the two plates are made of not metallic, preferably plastic, material.

Other advantages, features and use modes of the present invention will result evident from the following detailed description of some embodiments, shown by way of example and not for limitative purpose.

### Brief description of the figures

The enclosed figures will be referred to, wherein:
- Figure 1 shows a plan view of a first preferred embodiment of an applicator device according to the present invention;
- Figure 2 shows a plan view of the applicator device of Figure 1 in combination with a supporting element;
- Figures 3A and 3B show a top and bottom perspective view, respectively, of a second preferred embodiment of an applicator device according to the present invention in an exploded configuration;

- Figures 4A and 4B show a top and bottom perspective view, respectively, of the second preferred embodiment of an applicator device according to the present invention in combination with a supporting element in an exploded configuration;
- Figures 5A and 5B show a front and rear perspective view, respectively, of the second preferred embodiment of an applicator device according to the present invention in an exploded configuration, according to an attitude suitable to the anus-rectum-vagina-vulva treatment, with connecting means suitable to allow the anchoring to the patient's skin;
- Figures 6A e 6B show a front and rear perspective view, respectively, of the second preferred embodiment of an applicator device according to the present invention in combination with a supporting element, in an exploded configuration, according to an attitude suitable to the treatment of prostate, in particular anchored to a stepper or to a support for ultrasound apparatus;
- Figure 7 shows a plan view of a third preferred embodiment of an applicator device according to the present invention.

The above-mentioned Figures are to be meant exclusively with exemplifying and not limiting purposes.

### Detailed description of preferred embodiments

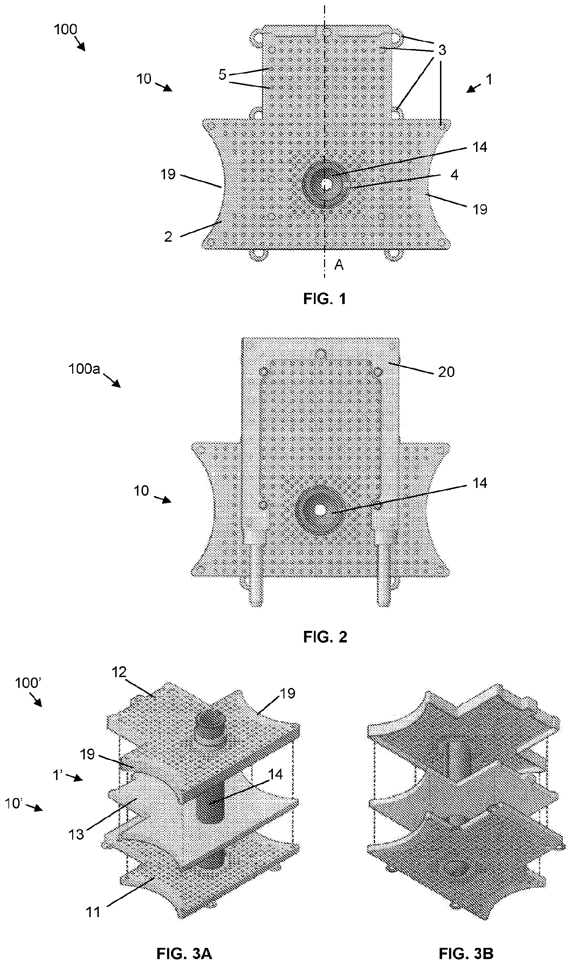

By referring to Figure 1, a first preferred embodiment of an applicator device for guiding the positioning of needle-like elements, such as needles or catheters, during the percutaneous insertion thereof is designated as a whole with 10. In particular, the device of the invention is suitable to perform intervention radiotherapy treatments, intervention/diagnostic procedures and diagnostic *imaging* on a patient.

The applicator device 10 comprises a main body 1 preferably shaped like a plate.

The plate-like shape is meant to designate a shape wherein two spatial sizes (defined hereinafter as main sizes) are predominant with respect to a third one, which third spazial size in the subject description is meant to coincide with the thickness of the main body 1.

In particular, the body 1 comprises an operating surface 2 which develops according to the two main sizes of the body 1, which can be seen in the plan view in Figure 1. Of course, in the plan view the thickness of the main body 1 cannot be seen.

Such surface 2 is defined as an operating surface because in use it corresponds to the face of the applicator wherein the needles or catheters are inserted in input, which needles or catheters cross the applicator until outgoing from an opposite face of the applicator with respect to the operating surface itself, in order to proceed with the percutaneous insertion.

The operating surface 2 can have a planar development, preferably according to a plane orthogonal to the thickness of the body 1, as shown in the preferred illustrated embodiment.

The main body 1 comprises at least two peripheral edge portions 19 which have a curved, preferably concave, profile to adjust to the anatomical shape of a user's leg, in particular to the thigh attachment.

The main body 1 has a substantially T-shaped overall profile seen in plan, wherein each one of said peripheral edge portions 19 is located at a respective end portion of a T arm. In the plan view of Figure 1 it is possible to see the concavity of the portions of peripheral edge 19.

The concave surfaces 19 result to be mutually specular with respect to an axis of symmetry A and they have an equal extension. According to preferred embodiments of the device, the axis of symmetry A corresponds to an axis of symmetry of the main body 1, which preferably extends in the main development direction of the T stem and it is orthogonal to the T arms, as still shown in Figure 1.

In order to allow the applicator device 10 to fulfil the guiding function for the percutaneous insertion of needles and catheters, the main body 1 further comprises a plurality of through holes 5, having a diameter indeed compatible for inserting needles or catheters.

In particular, the operating surface 2 has the plurality of through holes 5, which develop through the whole thickness of the plate, preferably mutually aligned according to a direction orthogonal to the operating surface 2. The holes are configured to allow the insertion and to guide the percutaneous penetration of needles or catheters, therefore they have a larger diameter than that of the elements to be guided.

According to particularly preferred embodiments of the invention, the applicator further has a through seat 4 configured for the insertion of a tubular guide element 14, in particular an endo-rectal cylinder, preferably eco-transparent. The endo-rectal cylinder 14 in turn is apt to receive a probe, for example an ultrasound probe, for guiding such probe within a patient's body district. The set of the applicator device 10 and the tubular guiding element 14 is designated as applicator assembly 100.

The applicator 10 preferably comprises means for locking, in particular locking reversibly, the guiding element 14 in the seat 4.

Preferably, the seat 4 has a development direction substantially orthogonal to the operating surface 2 of the main body 1, for example parallelly to the development direction of the plurality of holes 5.

Preferably, the seat 4 is positioned at the axis of symmetry A, still more preferably centred with respect to the two concave portions of peripheral edge 19, as shown in Figure 1.

According to alternative embodiments of the invention, the tubular guiding element 14 is implemented integrally to the main body 1, or it is fixed to the main body 1 permanently. The tubular guiding element could have different length and sizes or it could be inserted partially so as to modulate the length to adapt the applicator to the different vaginal or rectal anatomical situations.

According to a particularly advantageous aspect of the invention, the holes 5 are arranged with increasing density, that is a greater number of holes per surface unit, towards the seat 4, in other words they are arranged with density decreasing upon increasing of the distance from such seat 4.

According to alternative embodiments of the invention, on the operating surface 2 at least two regions with different distribution density of holes 5 can be defined, such as a first central region (in particular at or near the seat 4) having a first density of holes and a second peripheral region (substantially corresponding to the remaining portion of the operating surface 2) having a second density of holes, smaller than the first density.

The main body 1, as the whole applicator 10, is wholly made of not metallic material, preferably of plastics, or however it is made of a material suitable to allow to perform imaging diagnostic examinations (ultrasound, Magnetic Resonance - MR, Computerised Tomography - CT) without producing artefacts or being dangerous for the patient.

Additional preferred feature of an applicator 10 according to the present invention is the presence of means 3 for connecting to the patient's body district or to outer devices. For example, the connecting means 3 comprises seats for means for anchoring to supports of imaging apparatuses, steppers or supports for ultrasound apparatus. Preferably, all connection means comprised or applied to the device of the invention are made of not metallic material, for example plastics.

Such seats 3 can be made in the form of through holes, rings or eyelets configured for applying stitches (for connecting the applicator to the patient's skin) or fixing screws (for connecting applicator to outer apparatuses), preferably positioned on the peripheral profile of the main body 1 and/or at the end vertexes thereof. Preferably, the seats implemented as through holes are configured to allow even the insertion of a needle/catheter inside thereof.

Still, the connecting means 3 can allow to fix the applicator 10 to a supporting element 20, the latter configured to allow to fix the applicator device 10 thereto it is connected to an outer instrument or device, for example an imaging device. 1. In particular, the supporting element or the fixing support 20 is configured to be superimposed on the applicator device 10 at the operating surface 2 and fixed thereto by the connection means 3.

According to additional embodiments, the portions of the main body 1 at the T arms and the portion at the T stem could have different thicknesses to favour the hooking of the applicator 10 to other devices already present on the market, above all for the connection to steppers or supports for ultrasound apparatus. The thicknesses will be sufficient to guarantee the perpendicular insertion of the needle with respect to the surface of the applicator and the hooking of the T longitudinal stem to the above-mentioned anchoring devices.

By way of example, Figure 2 shows an applicator assembly 100a comprising, apart from the applicator device 10 and the tubular guiding element 14, a supporting element 20. All devices and elements comprised in the applicator assembly 100a preferably are made of not metallic material, for example plastics. In some situations, in order to guarantee a better stability, the element 20 could be made of metallic material.

A second preferred embodiment of the applicator device of the invention is shown in exploded configuration in Figures 3A and 3B, wherein it is designated with the numeral reference 10'. The set of the applicator device 10' and the tubular guiding element 14 is designated as a whole as applicator assembly 100'.

As it is clear from the Figures, the overall configuration of the device 10' still preferably is the one of a T-shaped plate, having two concave portions of peripheral edges 19 at the T arms.

Hereinafter, the second preferred embodiment of the applicator 10' will be described exclusively referring to the differences existing with respect to the already described embodiment.

The device 10' has a main body 1' apt to implement a system for locking by friction the needle-like elements inserted therein.

The main body 1' to this purpose has a modular configuration, comprising several mutually superimposable substantially plate-like elements, so that the overall thickness of the main body 1', and at last that of the applicator 10', is equal to the sum of the thicknesses of the plate-like elements.

The modular main body 1' comprises two outer bodies 11 and 12 made of stiff material (that is which cannot be perforated by the needle-like elements) and an intermediate body 13 which can be interposed therebetween, made of soft material (that is which can be perforated by the needle-like elements), preferably made of rubber.

Both outer bodies 11, 12 have the plurality of through holes 5 for the insertion of the needle-like elements, which through holes 5 match in an assembly configuration of the main body 1', in order to allow the simultaneous passage of the needle-like elements through both outer bodies 11, 12, so that they can cross the whole applicator 10'.

The two outer bodies 11, 12 are fixed to each other by dedicated locking means, for example by clamping threaded elements, to lock therebetween the intermediate body 13, preferably reversibly.

In use, each needle-like element is inserted in a respective hole of an outer body, it penetrates through the intermediate body and at last it goes out of the applicator by means of a corresponding through hole of the additional outer body.

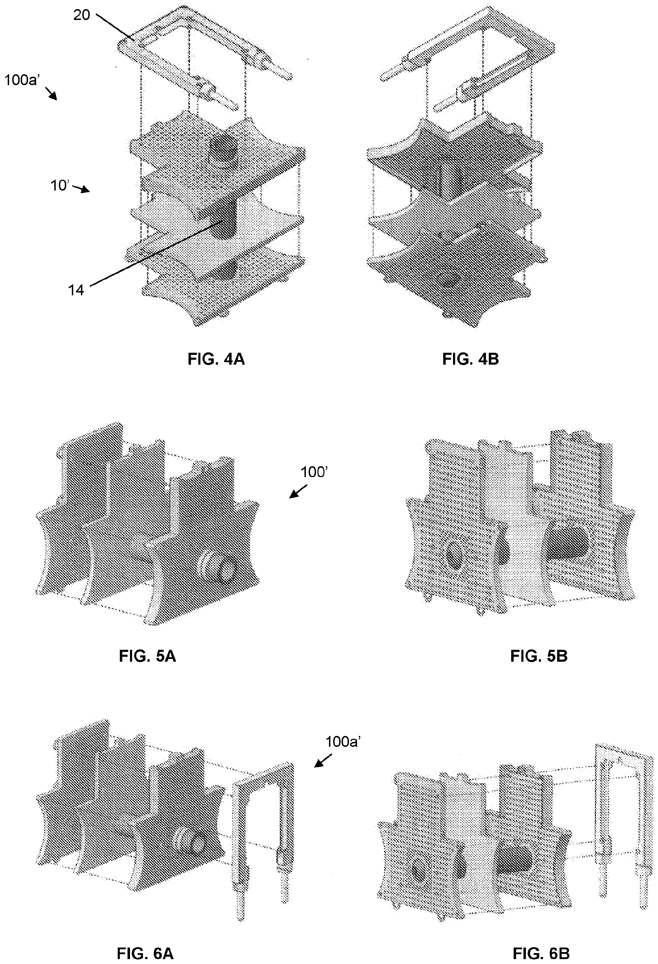

By referring to Figures 4A and 4B, an applicator assembly 100a' is shown, comprising an applicator device 10', a supporting element 20 and a tubular guiding element 14, all made of not metallic material, for example plastics.

Still, the couples of Figures 5A, 5B and 6A, 6B, respectively, show preferred configurations for using an applicator assembly 100' and 100a' according to the present invention, in particular Figures 5A and 5B show a suitable attitude for the treatment of the body districts of anus-vagina-vulva and Figures 6A and 6B show a suitable attitude for the treatment of the prostate.

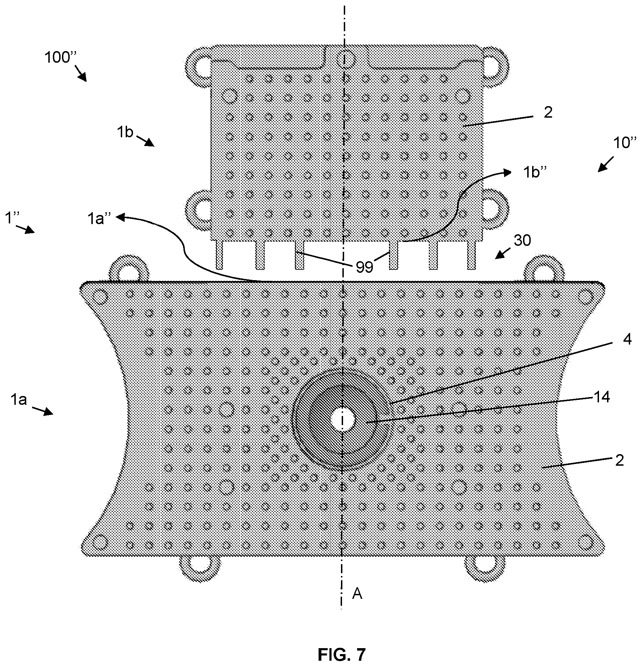

By referring to Figure 7, a third embodiment of the applicator device according to the present invention, designated as a whole with 10", will be now described and shown together with an applicator assembly 100" which comprises even a tubular guiding element 14 housed within the seat 4 of the applicator 10".

Such embodiment will be described exclusively relating to the differences existing with respect to the already described embodiments.

The applicator 10" has a main body 1" still overall shaped like a T, but having adjustable plan dimensions, as it comprises a plurality of selectively removable parts or portions.

Thanks to its modularity, the applicator 10" results to be more versatile with respect to the already treated variants, as the overall dimensions of the main body 1" can be adapted depending upon the body district to be treated. Therefore, the use thereof is particularly advantageous even within procedures involving particularly narrow anatomical areas, such as portions of vulva, vagina, anus or rectum, wherein the T-like configuration could result difficult to be applied. Under such circumstances, the use of one single portion of the main body 1" would make the treatment easier.

According to additional embodiments, the various portions of the main body 1" could have different thicknesses to favour the hooking of the applicator 10" to other devices, above all for the connection to steppers or supports for ultrasound apparatuses.

By referring to Figure 7, the main body 1" comprises at least two portions 1a and 1b, each one thereto a portion of an operating surface corresponds. Such portions 1a and 1b can be reversibly connected at least at a respective connecting region 1a" and 1b", which preferably corresponds to a respective peripheral portion when the applicator is plan seen.

According to the preferred embodiment the present description relates to, the two portions 1a and 1b correspond to the portion comprising the T arms and the portion of T stem, respectively.

At the two connecting regions 1a" and 1b" coupling means 30 is provided, apt to realize a reversible mechanical connection therebetween. For example, the coupling means 30 can include shaped profiles to implement a shape coupling between the two regions 1a" and 1b", in addition or in alternative to seats for the application of fixing means, preferably threaded means, such as screws or bolts, apt to implement and/or stabilize the coupling between the portions 1a and 1b.

In particular, according to the preferred embodiment shown in Figure 7, projecting elements or pins 99 are provided at the region 1b", whereas at the portion 1a" for each pin 99 a respective seat apt to receive it completely (not visible in Figure) is obtained, which to this purpose has a geometry substantially complementary thereto.

As it can be seen in the enclosed Figure, the pins 99 (and then their seats) can be arranged specularly with respect to an axis of symmetry A.

Preferably, the pins 99 implement a forcedly fixed connection with the respective seats, which then are slightly undersized; however, the insertion is possible thanks to the elastic deformability of pins and seats which, actually, implement a connection by friction.

The overall configuration is so that, when the two regions 1a" and 1b" are approached, each pin 99 inserts within its seat. The coupling is completed when the portions 1a and 1b abut on each other.

When the two portions 1a and 1b are in a completed coupling configuration, the main body 1" has an overall T-like shape, according to what already described with reference to the already described embodiments.

The present invention has been sofar described with reference to preferred embodiments, subjected to be combined whenever possible. It is to be meant that other embodiments belonging to the same inventive core may exist, as defined by the protective scope of the here below reported claims.

## Claims

1. An applicator device (10; 10'; 10") for interventional radiotherapy treatments (brachytherapy), general intervention and/or diagnostic procedures on a patient, comprising:
a plate-shaped main body (1; 1'; 1") comprising two peripheral edge portions (19) having a concave contour configured to abut on a patient's leg,
said peripheral edge portions (19) being respectively mirrored with respect to an axis of symmetry (A),
wherein said main body (1; 1'; 1") comprises a through seat (4) configured to house a tubular guiding element (14) in turn configured to receive a probe, such as an ultrasound probe, and to guide it within a patient's body region,
**characterized in that** said main body (1; 1'; 1") comprises a plurality of through holes (5) configured to accommodate needle-like elements, the density of said plurality of holes (5) decreasing with the distance from said seat (4).

2. The applicator device (10; 10'; 10") according to claim 1, wherein said main body (1; 1'; 1') has a substantially T-shaped overall profile, wherein each one of said peripheral edge portions (19) is located at a respective end portion of a T arm.

3. The applicator device (10; 10'; 10") according to claim 1 or 2, wherein said main body (1; 1'; 1") has an operating surface (2) having substantially planar configuration, said operating surface (2) being the surface of the applicator wherein the needles or catheters are inserted when in use.

4. The applicator device (10; 10'; 10") according to any one of the preceding claims, wherein said main body (1; 1'; 1") is made of plastic material.

5. The applicator device (10; 10'; 10") according to one of the preceding claims, wherein said seat (4) has a development direction substantially orthogonal to an operating surface (2) of said main body (1; 1'; 1"), said operating surface (2) being the surface of the applicator wherein the needles or catheters are inserted when in use.

6. The applicator device (10; 10'; 10") according to claim 5, wherein said seat (4) is positioned at said axis of symmetry (A) centred with respect to said two peripheral edge portions (19).

7. The applicator device (10') according to any one of the preceding claims, wherein said main body (1') has a modular sandwich configuration, comprising a first (11) and a second outer body (12), each one having a plurality of through holes (5) configured to house needle-like elements, and an intermediate body (13) interposed between said first (11) and second outer body (12) and made of material configured to be perforated by the needle-like elements.

8. The applicator device (10") according to any of the preceding claims, wherein said main body (1") comprises a first (1a) and a second portion (1b), to each one thereof a portion of an operating surface (2) corresponds, which can be reversibly connected at least at a respective connection region (1a", 1b"), wherein at said connection regions (1a", 1b") coupling means (30), configured to realize a reversible mechanical connection between said first (1a) and second portion (1b), are provided.

9. An applicator assembly (100; 100'; 100a; 100a'; 100") for interventional radiotherapy treatments (brachytherapy), general intervention and/or diagnostics procedures on a patient, comprising:
an applicator device (10; 10'; 10") according to any of the preceding claims, a fixing support (20) configured to be connected to said applicator device (10,10') and to an imaging device.

10. The applicator assembly (100; 100'; 100a; 100a'; 100") according to claim 9, or the applicator device (10; 10'; 10") according to any of claims 1-8, wherein said tubular guide element (14) is made of eco-transparent material.

11. The applicator assembly (100a; 100a') according to claims 9 or 10, wherein said fixing support (20) is configured to be superimposed on said applicator device (10; 10') at an operating surface (2), said operating surface (2) being the surface of the applicator wherein the needles or catheters are inserted when in use.

## Patentansprüche

1. Applikationsvorrichtung (10; 10'; 10") für interventionelle Strahlentherapiebehandlungen (Brachytherapie), allgemeine Eingriffe und/oder diagnostische Verfahren an einem Patienten, umfassend:
einen plattenförmigen Hauptkörper (1; 1'; 1"), der zwei Umfangsrandabschnitte (19) mit einer konkaven Kontur umfasst, die so ausgestaltet sind, dass sie an einem Bein des Patienten anliegen,
wobei die Umfangsrandabschnitte (19) jeweils in Bezug auf eine Symmetrieachse (A) gespiegelt sind,
wobei der Hauptkörper (1; 1'; 1") einen durchgehenden Sitz (4) umfasst, der so ausgestaltet ist, dass er ein röhrenförmiges Führungselement (14) aufnimmt, welches wiederum so ausgestaltet ist, dass es eine Sonde, wie zum Beispiel eine Ultraschallsonde, aufnimmt und sie innerhalb eines Körperbereichs eines Patienten führt,
**dadurch gekennzeichnet, dass** der Hauptkörper (1; 1'; 1") eine Vielzahl von Durchgangslöchern (5) umfasst, die so ausgestaltet sind, dass sie nadelartige Elemente beherbergen, wobei die Dichte der Vielzahl von Löchern (5) mit dem Abstand von dem Sitz (4) abnimmt.

2. Applikationsvorrichtung (10; 10'; 10") nach Anspruch 1, wobei der Hauptkörper (1; 1'; 1') ein im Wesentlichen T-förmiges Gesamtprofil hat, wobei jeder der Umfangsrandabschnitte (19) an einem jeweiligen Endabschnitt eines T-Arms angeordnet ist.

3. Applikationsvorrichtung (10; 10'; 10") nach Anspruch 1 oder 2, wobei der Hauptkörper (1; 1'; 1") eine Arbeitsfläche (2) mit im Wesentlichen planarer Konfiguration hat, wobei die Arbeitsfläche (2) die Fläche des Applikators ist, in die die Nadeln oder Katheter bei der Verwendung eingeführt werden.

4. Applikationsvorrichtung (10; 10'; 10") nach einem der vorhergehenden Ansprüche, wobei der Hauptkörper (1; 1'; 1") aus einem Kunststoffmaterial hergestellt ist.

5. Die Applikationsvorrichtung (10; 10'; 10") nach einem der vorhergehenden Ansprüche, wobei der Sitz (4) eine Entwicklungsrichtung aufweist, die im Wesentlichen orthogonal zu einer Arbeitsfläche (2) des Hauptkörpers (1; 1'; 1") ist, wobei die Arbeitsfläche (2) die Fläche des Applikators ist, in die die Nadeln oder Katheter bei der Verwendung eingeführt werden.

6. Applikationsvorrichtung (10; 10'; 10") nach Anspruch 5, wobei der Sitz (4) an der Symmetrieachse (A) zentriert in Bezug auf die beiden Umfangsrandabschnitte (19) angeordnet ist.

7. Applikationsvorrichtung (10') nach einem der vorhergehenden Ansprüche, wobei der Hauptkörper (1') eine modulare Sandwich-Konfiguration hat, die einen ersten (11) und einen zweiten Außenkörper (12) umfasst, die jeweils eine Vielzahl von Durchgangslöchern (5) haben, die so ausgestaltet sind, dass sie nadelartige Elemente aufnehmen können, und einen Zwischenkörper (13), der zwischen dem ersten (11) und dem zweiten Außenkörper (12) angeordnet ist und aus einem Material hergestellt ist, das so ausgestaltet ist, dass es von den nadelartigen Elementen durchstochen werden kann.

8. Applikationsvorrichtung (10") nach einem der vorhergehenden Ansprüche, wobei der Hauptkörper (1") einen ersten (1a) und einen zweiten Abschnitt (1b) umfasst, denen jeweils ein Abschnitt einer Arbeitsfläche (2) entspricht, der zumindest an einem jeweiligen Verbindungsbereich (1a", 1b") reversibel verbunden werden kann, wobei an den Verbindungsbereichen (1a", 1b") Kopplungsmittel (30) vorgesehen sind, die so ausgestaltet sind, dass sie eine reversible mechanische Verbindung zwischen dem ersten (1a) und zweiten Abschnitt (1b) verwirklichen.

9. Applikationsanordnung (100; 100'; 100a; 100a'; 100") für interventionelle Strahlentherapiebehandlungen (Brachytherapie), allgemeine Eingriffe und/oder diagnostische Verfahren an einem Patienten, umfassend:
eine Applikationsvorrichtung (10; 10'; 10") nach einem der vorhergehenden Ansprüche, eine Befestigungshalterung (20), die so ausgestaltet ist, dass sie mit der Applikationsvorrichtung (10, 10') und mit einer Bildgebungsvorrichtung verbunden werden kann.

10. Die Applikationsanordnung (100; 100'; 100a; 100a'; 100") nach Anspruch 9, oder die Applikationsvorrichtung (10; 10'; 10") nach einem der Ansprüche 1-8,
wobei das röhrenförmige Führungselement (14) aus transparentem Ökomaterial hergestellt ist.

11. Applikationsanordnung (100a; 100a') nach Anspruch 9 oder 10, wobei die Befestigungshalterung (20) so ausgestaltet ist, dass sie die Applikationsvorrichtung (10; 10') an einer Arbeitsfläche (2) überlagert, wobei die Arbeitsfläche (2) die Oberfläche des Applikators ist, in die die Nadeln oder Katheter bei der Verwendung eingeführt werden.

## Revendications

1. Dispositif applicateur (10 ; 10'; 10") pour des traitements de radiothérapie interventionnelle (curiethérapie), procédures d'intervention générale et/ou de diagnostic sur un patient, comprenant :
un corps principal (1 ; 1' ; 1") en forme de plaque comprenant deux parties de bord périphérique (19) ayant un contour concave configuré pour venir en butée sur une jambe du patient,
lesdites parties de bord périphérique (19) étant respectivement spéculaires par rapport à un axe de symétrie (A),
dans lequel ledit corps principal (1 ; 1' ; 1") comprend un siège débouchant (4) configuré pour loger un élément de guidage tubulaire (14) configuré à son tour pour recevoir une sonde, telle qu'une sonde à ultrasons, et pour la guider dans une région corporelle du patient,
**caractérisé en ce que** ledit corps principal (1 ; 1' ; 1") comprend une pluralité de trous débouchants (5) configurés pour loger des éléments en forme d'aiguille, la densité de ladite pluralité de trous (5) diminuant avec la distance par rapport audit siège (4).

2. Dispositif applicateur (10; 10'; 10") selon la revendication 1, dans lequel ledit corps principal (1 ; 1' ; 1") a un profil global sensiblement en forme de T, dans lequel chacune desdites parties de bord périphérique (19) est positionnée au niveau d'une partie d'extrémité respective d'un bras en T.

3. Dispositif applicateur (10 ; 10' ; 10") selon la revendication 1 ou 2, dans lequel ledit corps principal (1 ; 1' ; 1") a une surface opérationnelle (2) ayant une configuration sensiblement planaire, ladite surface opérationnelle (2) étant la surface de l'applicateur, dans laquelle les aiguilles ou les cathéters sont insérés lors de l'utilisation.

4. Dispositif applicateur (10 ; 10' ; 10") selon l'une quelconque des revendications précédentes, dans lequel ledit corps principal (1 ; 1' ; 1") est réalisé à partir de matière plastique.

5. Dispositif applicateur (10; 10'; 10") selon l'une des revendications précédentes, dans lequel ledit siège (4) a une direction de développement sensiblement orthogonale à une surface opérationnelle (2) dudit corps principal (1 ; 1' ; 1"), ladite surface opérationnelle (2) étant la surface de l'applicateur, dans laquelle les aiguilles ou les cathéters sont insérés lors de l'utilisation.

6. Dispositif applicateur (10 ; 10'; 10") selon la revendication 5, dans lequel ledit siège (4) est positionné audit axe de symétrie (A) centré par rapport auxdites deux parties de bord périphérique (19).

7. Dispositif applicateur (10') selon l'une quelconque des revendications précédentes, dans lequel ledit corps principal (1') a une configuration de sandwich modulaire, comprenant un premier (11) et un second corps externe (12), ayant chacun une pluralité de trous débouchants (5) configurés pour loger des éléments en forme d'aiguille, et un corps intermédiaire (13) intercalé entre ledit premier (11) et le second corps externe (12) et réalisé avec un matériau configuré pour être perforé par les éléments en forme d'aiguille.

8. Dispositif applicateur (10") selon l'une quelconque des revendications précédentes, dans lequel ledit corps principal (1") comprend une première (1a) et une seconde partie (1b), à chacune desquelles correspond une partie d'une surface opérationnelle (2) qui peut être raccordée, de manière réversible, au moins à une région de raccordement (1a", 1b") respective, dans lequel au niveau desdites régions de raccordement (1a", 1b"), on prévoit des moyens de couplage (30) configurés pour réaliser un raccordement mécanique réversible entre ladite première (1a) et seconde partie (1b).

9. Ensemble applicateur (100 ; 100' ; 100a ; 100a' ; 100") pour des traitements de radiothérapie interventionnelle (curiethérapie), des procédures d'intervention générale et/ou de diagnostic sur un patient, comprenant :
un dispositif applicateur (10 ; 10' ; 10") selon l'une quelconque des revendications précédentes,
un support de fixation (20) configuré pour être raccordé audit dispositif applicateur (10, 10') et à un dispositif d'imagerie.

10. Ensemble applicateur (100 ; 100' ; 100a ; 100a' ; 100") selon la revendication 9 ou dispositif applicateur (10 ; 10'; 10") selon l'une quelconque des revendications 1 à 8, dans lequel ledit élément de guidage tubulaire (14) est réalisé avec un matériau éco-transparent.

11. Ensemble applicateur (100a; 100a') selon les revendications 9 ou 10, dans lequel ledit support de fixation (20) est configuré pour être superposé sur ledit dispositif applicateur (10 ; 10') au niveau d'une surface opérationnelle (2), ladite surface opérationnelle (2) étant la surface de l'applicateur dans laquelle les aiguilles ou les cathéters sont insérés lors de l'utilisation.
